# EUROPÄISCHE PATENTSCHRIFT

(11) **EP 1 106 205 B1**
(45) Veröffentlichungstag und Bekanntmachung des Hinweises auf die Patenterteilung: **26.03.2008**
(21) Anmeldenummer: 00125105.7
(22) Anmeldetag: 17.11.2000
(51) Int. Cl.: A61N 1/365

(54) **Kardiologisches Geräteimplantat mit Tag-Nacht-Wechsel Erkennung**
Implanted cardiac device with detection of day/night periods
Dispositif cardiaque implanté avec detection des phases jour/nuit

(30) Priorität: 06.12.1999 DE 19958735
(43) Veröffentlichungstag der Anmeldung: 13.06.2001
(73) Patentinhaber: BIOTRONIK GmbH & Co. KG, 12359 Berlin (DE)
(72) Erfinder: Meyer, Wolfgang, 91052 Erlangen (DE)
(74) Vertreter: Lindner-Vogt, Karin L.

(56) Entgegenhaltungen:
- WO-A-93/18821
- WO-A-99/30777

## Beschreibung

Die Erfindung betrifft ein kardiologisches Geräteimplantat, insbesondere einen Herzschrittmacher, zur Detektion eines signifikanten Ereignisses oder Zustandes, dem ein Patient unterworfen ist, wie eines Tag-Nacht-Wechsels, eines pathologischen Zustandes o. dgl.

Aus der WO 93/18821 A1 ist ein Impedanzmeßsystem zur Überwachung physiologischer Zustände von Patientengewebe bekannt. Die Vorrichtung umfasst Elektroden, die mit dem zu überwachenden Gewebe in Kontakt stehen, Schaltkreise zur Erzeugung elektrischer Signale bei zumindest zwei Frequenzen zur Einwirkung auf das Gewebe und Schaltkreise zur Erfassung der Impedanz des Gewebes. Es ist zusätzlich eine Einrichtung zur Erfassung von Veränderungen in der Beziehung der gemessenen Impedanzen und zur Verarbeitung der erfassen Veränderungen in der Impedanzbeziehung vorgesehen, um einen Hinweis auf die Gewebeeigenschaften zu erhalten. Die Vorrichtung ist bei einem implantierbaren Stimulator, wie beispielsweise einem Herzschrittmacher einsetzbar, bei dem die Pulsfrequenz als Funktion des erfassen Gewebezustandes variiert wird.

Die WO99/30777 A1 offenbart einen Herzschrittmacher, bei dem Impedanzen zwischen verschiedenen Herzregionen gemessen werden. Die Impedanzwerte werden weiterverarbeitet, um zu bestimmen, ob atriale oder ventrikuläre Rhythmen in Ordnung oder unphysiologisch sind. Diese Bestimmung kann beispielsweise dadurch vorgenommen werden, dass Unterschiede in den Impedanzwerten über die Zeit erfasst und diese Unterschiede nach vorbestimmten Kriterien verglichen werden. Falls Arhythmien festgestellt werden, kann der Herzschrittmacher einen entsprechenden Antwortpuls geben. Gleichzeitig werden die Quer-Messungen der Impedanzen beispielsweise zwischen rechtem Atrium und linkem Ventrikel oder zwischen linkem Atrium und rechtem Ventrikel gespeichert und so weiterverarbeitet, dass daraus bestimmt wird, ob ein Herzversagen droht. Falls ja, kann das Gerät eine Warnung ausgeben, die von einem externen Programmiergerät aufgenommen werden kann.

Zum Hintergrund der Erfindung ist festzuhalten, daß bei klinischen Studien im Zusammenhang mit Herzschrittmachern die unipolare intrakardiale Impedanz mit dem Zweck untersucht wurde, aus den Meßergebnissen Rückschlüsse auf eine mögliche Verbesserung der Steuermöglichkeiten von Herzschrittmachern ziehen zu können. Diese Untersuchungen wurden mit einem Herzschrittmacher vorgenommen, der entsprechende Impedanzwerte auf eine externe Überwachungseinrichtung übertrug. Dabei wurden anläßlich zweier Nachsorgeuntersuchungen jeweils sechs und zwölf Wochen nach der Implantation des Herzschrittmachers Daten über den Signalverlauf der gemessenen intrakardialen Impedanz über eine Zeitraum von 24 Stunden erfaßt und gespeichert. Aus dieser Messung der intrakardialen Impedanz wurde der morphologische Signalverlauf der Impedanz so erfaßt, daß die Impedanzkurve des Herzens während eines Herzschlages aufgezeichnet werden konnte. Wie im folgenden anhand des Ausführungsbeispieles noch näher erläutert wird, wurden gemeinsame Merkmale und Unterschiede der gemessenen Impedanzkurven bei den beiden Nachsorgeuntersuchungen mit statistischen Methoden analysiert. Zielrichtung war dabei, die gewonnenen Erkenntnisse für eine weitere Optimierung der Herzschrittmacherfunktionen, z. B. im Zusammenhang mit der Tag-/Nacht-Anpassung der Steuerparameter oder für die Erkennung pathologischer Unregelmäßigkeiten, wie die Anfälligkeit gegen Extrasystolen oder den plötzlichen Herztod, auszunützen.

Aus den klinischen Untersuchungen hat sich nun beispielsweise ergeben, daß die Morphologie der gemittelten unipolaren intrakardialen Impedanz über den Zeitraum der Nachsorgeuntersuchungen hinweg relativ stabil ist. Aufgrund der statistischen Auswertungen wurden Unterschiede bei der Korrelation der Meßwerte zwischen verschiedenen Patienten festgestellt, die zu diagnostischen Zwecken ausgenützt werden können. Schließlich können Unterschiede zwischen den durchschnittlichen Tages- und Nacht-Kurven ein Kriterium für eine einfache Tag-/Nacht-Unterscheidung und - Erkennung bieten.

Davon ausgehend schlägt die Erfindung ein kardiologisches Geräteimplantat, also insbesondere einen Herzschrittmacher vor, das bestimmte Verfahrensschritte zur Detektion eines signifikanten Ereignisses oder Zustandes aufweist, dem ein Patient unterworfen ist. Insoweit betrifft die Erfindung also keine Komplettsteuerung eines Herzschrittmachers, sondern den in der Zweckangabe angegebenen Teilaspekt. Diesem wiederum kann in der Implementierung eines bestimmten Steuerprogramms auf einem Herzschrittmacher eine entsprechende Funktion zugewiesen werden, die durch das Betriebsverfahren des erfindungsgemäßen Geräteimplantats erfüllt wird.

Laut Patentanspruch 1 weist dieses Betriebsverfahren folgende Verfahrensschritte auf:
- Messung der intrakardialen Impedanz des Herzens,
- Erfassung des morphologischen Signalverlaufs der Impedanz anhand mindestens eines morphologisch repräsentativen Parameters des Signalverlaufs,
- laufende Speicherung des Parameters aus einer definierten Meßperiode,
- Berechnung eines Korrelationswertes für den gespeicherten Parameter, und
- Vergleichen des Korrelationswertes mit einem Referenz-Korrelationswert, wobei das Überschreiten einer definierten Abweichung zwischen Korrelations- und Referenz-Korrelationswerten das Vorliegen des signifikanten Ereignisses indiziert.

Das vorstehende Auswerteverfahren wird erfindungsgemäß im Betrieb des kardiologischen Geräteimplantates dazu ausgenützt, einen Tag-/Nacht- oder Nacht-/Tag-Wechsel zu erkennen. Wenn beispielsweise Autokorrelationswerte durch Vergleich von sich um eine definierte Zeitverschiebung unterscheidenden Parameterdaten fortlaufend berechnet und ausgewertet werden, so kommt es bei einem Wechsel zwischen Tag und Nacht (oder umgekehrt) durch den sich dabei ändernden typischen Signalverlauf zu einer Erniedrigung des Autokorrelationswertes, der sich nach Durchlauf des Wechsels durch das Intervall wieder auf seinen Ausgangswert erhöht. Der Wechsel generiert also ein Minimum in der zeitlichen Folge der Autokorrelationswerte, das erkannt wird und zur Einstellung des Betriebsprogramms des kardiologischen Geräteimplantates von Tag- auf Nachtbetrieb anwendbar ist.

Das Verfahren setzt also auf die Messung der intrakardialen Impedanz des Herzens auf und erfaßt durch eine in der Regel unipolare Elektrode, die im Myokard des Herzens verankert ist, den morphologischen Signalverlauf der Impedanz, also nicht nur einen bestimmten Meßwert, sondern die Impedanz in Abhängigkeit von verschiedenen Zeitpunkten während eines Kontraktionszyklus des Herzens. Dabei können allerdings im Extremfall ein Parameter, vorzugsweise aber wohl zwei morphologische repräsentative Parameterdaten des Signalverlaufs, wie z. B. die Peak-to-Peak-Amplitude als Parameterdaten ausreichen. Diese Parameterdaten werden dann über eine definierte Meßperiode erfaßt, die mehrere Stunden des Tages - je nach Speicherkapazität in dem kardiologischen Geräteimplantat - dauern kann. Für diese gespeicherten Parameterdaten werden Korrelationswerte berechnet, wofür - je nach Funktion und Ausrichtung des Betriebsverfahrens - unterschiedliche Berechnungsverfahren herangezogen werden können. Insoweit ist unter "Korrelationswert" auch nicht ausschließlich die Berechnung einer Korrelation im streng statistischen Sinn gemeint. Es soll darunter im allgemeinen Sinn die Ermittlung aussagekräftiger Werte verstanden werden, wie die Berechnung der quadratischen Abweichung, der Standardabweichung, der Autokorrelation, der eigentlichen Korrelation im strengen Sinne, der Kreuz-Korrelation oder auch schlicht die Abweichung eines Signalverlaufes zu einem Norm- oder Referenzsignalverlauf. Dies wird im folgenden noch weiter vertieft werden.

Für die Zwecke der Erfindung werden schließlich die Korrelationswerte mit einem Referenz-Korrelationswert verglichen, wobei das Überschreiten einer definierten Abweichung zwischen diesen beiden Korrelationswerten das Vorliegen eines signifikanten Ereignisses indiziert.

Gemäß weiterer bevorzugter Ausführungsformen der Erfindung können die verschiedenen Korrelationswerte fortlaufend berechnet und mit den Referenz-Korrelationswerten verglichen werden. Dies bedeutet, daß eine fortlaufende Überwachung des Herzens auf etwaige Abweichungen von der Norm erfolgt, wobei vorzugsweise die während eines bestimmten Intervalls errechneten Korrelationswerte laufend zu Auswertungszwecken gespeichert werden können. Insgesamt erfolgt also eine Auswertung der Parameterdaten des Signalverlaufs, die auf ein bestimmtes Zeitfenster zugreift, das sich während des Betriebes des kardiologischen Geräteimplantates von der jeweils aktuellen Zeit um die Auswerte-Intervalldauer nach hinten erstreckt.

Gemäß einer weiteren bevorzugten Ausführungsform der Erfindung können möglicherweise pathologische Zustände des Herzens dadurch erkannt werden, daß die ermittelten Korrelationswerte analysiert und das Überschreiten einer definierten Abweichung von Referenz-Korrelationswerten als Vorliegen eines möglicherweise pathologischen Zustandes erkannt wird. Entsprechend kann das kardiologische Geräteimplantat dann ein Warnsignal setzen, das von außen detektierbar ist.

Da die einschlägigen klinischen Untersuchungen gezeigt haben, daß sich die Korrelationswerte in Abhängigkeit der Implantierungsdauer des Implantates signifikant ändern können, ist es entsprechend von Vorteil, die Referenz-Korrelationswerte abhängig von der Implantierungsdauer des Implantates einstellbar zu machen. Damit ist das im Geräteimplantat ablaufende Betriebsverfahren entsprechend dessen Einsatzdauer optimal angepaßt.

Eine besondere Ausführungsform der Erfindung zielt auf die statistische und spektrale Analyse der Parameterdaten des Signalverlaufs der durchschnittlichen intrakardialen Impedanz ab. Dabei braucht lediglich als Korrelationswert für die gespeicherten Parameter die Abweichung der Einzelsignalverläufe - also beispielsweise die quadratische oder Standardabweichung - von einem gemittelten Signalverlauf oder voneinander erfaßt und mit Mitteln der Spektralanalyse analysiert zu werden. Bei dieser Analyse werden regelmäßige wiederkehrende Abweichungen mit relativ kleinen Frequenzen, die mit physiologischen Funktionen des Körpers in noch näher zu erläuternder Weise gekoppelt sind, detektiert. Aufgrund dieser Detektion kann dann das Betriebsverfahren entsprechende Maßnahmen im Herzschrittmacher implementieren, die sich auf die detektierte Situation einstellen.

Gemäß einer weiteren bevorzugten Ausführungsform der Erfindung kann diese so ausgelegt sein, daß zwischen verschiedenen Auswerteverfahren und -parametern, wie sie in den vorstehenden Ansprüchen angegeben sind, ausgewählt werden kann. Diese Auswahl kann durch entsprechende Implementierung des Betriebsprogramms automatisch in Abhängigkeit des vorhergehenden Auswerteergebnisses erfolgen. Auch eine reine Vorgabe durch das Betriebsprogramm selbst ohne Bezugnahme auf die vorherigen Auswerteergebnisse ist möglich.

Im folgenden werden die der Erfindung zugrundeliegenden wissenschaftlichen Erkenntnisse und das darauf beruhende Betriebsverfahren für einen Herzschrittmacher in seinen verschiedenen Varianten anhand der beigefügten Zeichnungen näher erläutert. Es zeigen:
- Fig. 1: ein Kurvendiagramm des gemittelten Impedanz-Signalverlaufs mit einer Mittelungszeit über den gesamten Tagesverlauf,
- Fig. 2: ein Kurvendiagramm analog Fig. 1 mit einer Mittelungszeit nur über die Tagesstunden,
- Fig. 3: ein Kurvendiagramm analog Fig. 1 und 2 mit einer Mittelungszeit nur über die Nachtstunden,
- Fig. 4: eine Tabelle der Kreuzkorrelationsdaten zwischen der Gesamtmittelwertkurve, Tagesmittelwertkurve und Nachtmittelwertkurve verschiedener Patienten zu einem Zeitpunkt von sechs Wochen nach der Implantation eines Herzschrittmachers,
- Fig. 5: eine Tabelle analog Fig. 4 der Kreuzkorrelationsdaten nach drei Monaten nach der Implantation des Herzschrittmachers,
- Fig. 6 und 7: Kurvendiagramme des Verlaufes der Standardabweichung der Tages- und Nachtmittelwertkurven für verschiedene Patienten nach sechs Wochen bzw. drei Monate nach der Implantation des Herzschrittmachers, und
- Fig. 8: ein Kurvendiagramm zur Darstellung eines Ausschnittes des zeitlichen Verlaufes des linearen Korrelationskoeffizienten eines Patienten nach sechs Wochen nach der Implantation des Herzschrittmachers.

Die der Erfindung zugrundeliegende klinische Studie basiert auf der Nachsorgeuntersuchung von acht Patienten, die sechs Wochen bzw. drei Monate nach der Einpflanzung des Herzschrittmachers durchgeführt wurde. Mit Hilfe einer unipolaren Elektrode wurde über 24 Stunden hinweg die intrakardiale Impedanz des Herzens mit einem Herzschrittmacher des Typs "INOS" der Anmelderin durchgeführt. Der Herzschrittmacher war auf den DDD-Modus programmiert. Die von der Elektrode ermittelten Meßkurven wurden mit einem "Unilyzer-Holter-Überwachungsgerät" erfaßt. Jede Signalkurve während eines Herzschlages wurde aufgelöst in 32 Meßpunkte aufgezeichnet und gespeichert. Bei diesen Messungen wurde basierend auf einem sehr groben Ansatz zur Nachterkennung mit Hilfe der zirkadianen Variation eines Variabilitätsmaßes des intrakardialen Impedanzsignales eine grobe Tag-/Nacht-Unterscheidung durchgeführt.

Auf dieser Basis wurden bei den durchgeführten Messungen alle Signalkurven der Patienten jeweils über den Gesamttag, nur über die Tagesstunden und nur über die Nachtstunden gemittelt, und der durchschnittliche morphologische Signalverlauf mit der lokalen Standardabweichung in jedem Meßpunkt aller Kurven bestimmt und die Korrelation der unterschiedlichen Paare von Kurven ermittelt.

Im einzelnen wurden ferner bestimmt:
- Die Korrelation der Gesamtmittelwert-, Tagesmittelwert- und Nachtmittelwertkurven im Vergleich zwischen sechs Wochen und drei Monaten nach Implantation,
- die Kreuzkorrelationswerte dieser drei Kurven zwischen verschiedenen Patienten zu den angegebenen Zeitpunkten,
- die Kreuzkorrelation von Tagesmittelwert und Nachtmittelwert jedes Patienten bei sechs Wochen und drei Monaten nach der Implantation,
- die Peak-to-Peak-Amplitude (Differenz zwischen Maximalwert und Minimalwert) der Gesamtmittelwert-, Tagesmittelwert- und Nachtmittelwertkurven, und
- der Verlauf und der Mittelwert der Standardabweichung der Mittelwertskurven.

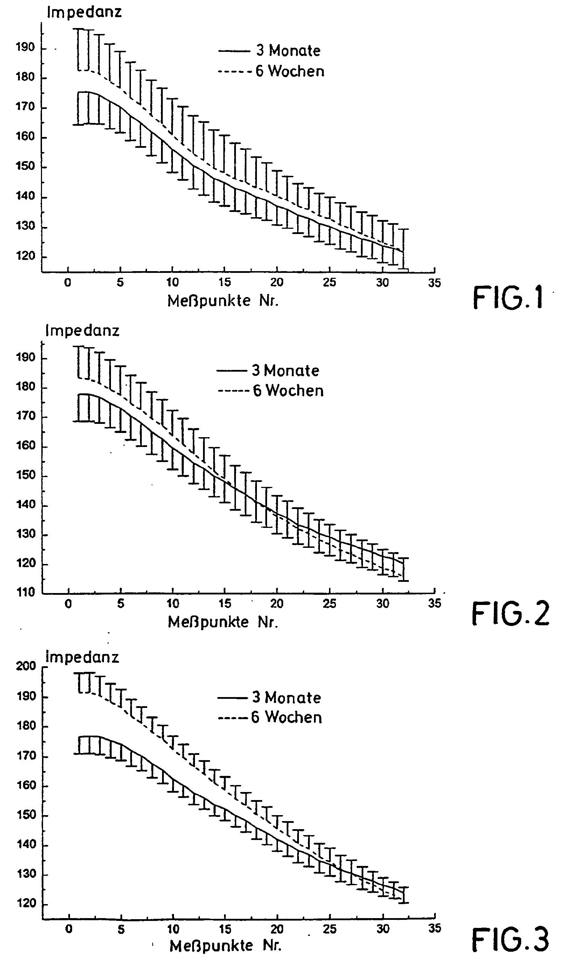

Fig. 1 spiegelt nun einen typischen morphologischen Signalverlauf der kardialen Impedanz während eines Halbzyklusses des Herzens wieder. In diesem Diagramm - wie auch in den Fig. 2 und 3 - sind auf der x-Achse die 32 Meßpunkte zur Auflösung des morphologischen Signalverlaufs aufgetragen. Auf der y-Achse ist in willkürlichen Einheiten die intrakardiale Impedanz aufgetragen. Die strichlierte Linie in den Diagrammen stellt den morphologischen Signalverlauf bei den Messungen sechs Wochen nach der Implantation, die durchgezogene Linie drei Monate nach der Implantation dar. Die vertikalen Balken geben die Standardabweichung dieser Mittelwerte wieder. Die resultierenden Morphologien nach sechs Wochen und drei Monaten des in den Fig. 1 bis 3 repräsentierten typischen Patienten geben Anlaß für folgende Feststellungen:
- Zwischen sechs Wochen und drei Monaten wird die Amplitude der gemittelten Impedanzkurve gedämpft, d. h. es wird der Unterschied zwischen dem Maximal- und Minimalwert innerhalb des Meßfensters erniedrigt.
- Der Dämpfungseffekt auf die Gesamtmittelwertkurve scheint sich hauptsächlich aufgrund des Dämpfens der Nachtkurve zu ergeben.
- Die qualitative Morphologie ist während der Zeit der Nachsorgeuntersuchung relativ stabil.
- Die qualitative Morphologie hängt wenig vom Tag-/Nacht-Unterschied ab.

Berechnet man nun aus den Meßwerten verschiedene Korrelationswerte, so lassen sich folgende Ergebnisse ermitteln.

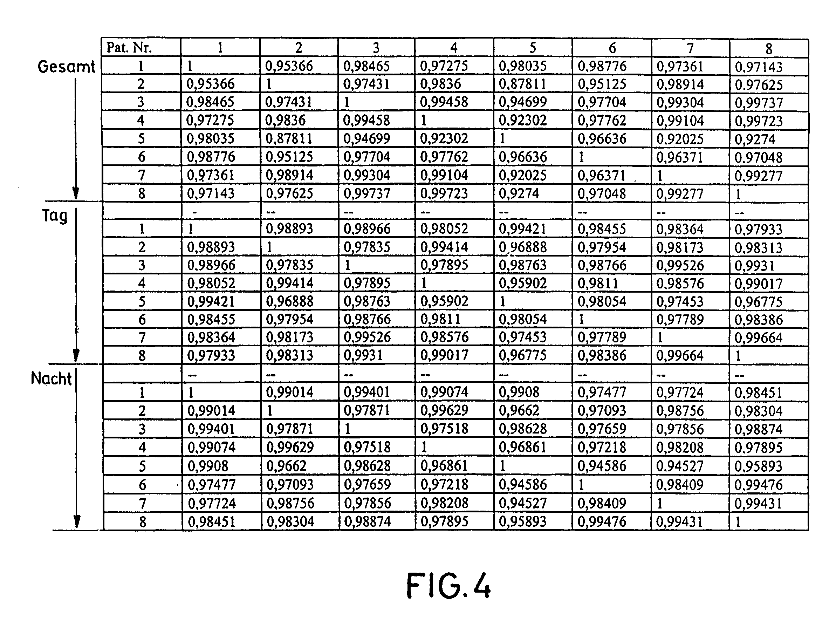

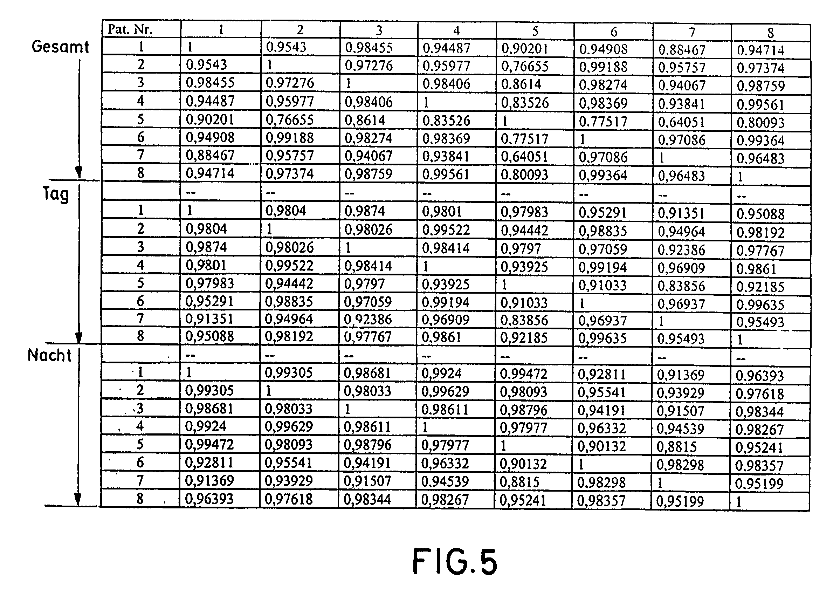

Der lineare Korrelationskoeffizient jeder Mittelwertkurve verglichen zu sich selbst bei sechs Wochen und drei Monaten ist ein direkter Parameter, um Veränderungen in der Morphologie zu detektieren. Wie aus den Tabellen gemäß Fig. 4 und 5 hervorgeht, sind die Korrelationen zwischen den Gesamtmittelwerten bei sechs Wochen und drei Monaten nur bei den Patienten 5, 6 und 7 etwas stärker von 1.0 abweichend als bei den verbleibenden Patienten. Auch die separaten Durchschnittskurven für Tag und Nacht zeigen eine gute Korrelation zwischen sechs Wochen und drei Monaten. Insofern haben die Durchschnittskurven eine extreme Stabilität bezüglich langfristiger Änderungen. Vergleicht man die jeweiligen Kurven zwischen verschiedenen Patienten, sollte sich eine größere Variabilität als bei Vergleichen innerhalb eines Patienten ergeben, was einem geringerem Korrelationskoeffizienten entspricht. Dieser Effekt kann zeitabhängig sein, wie sich aus den Kreuzkorrelationskoeffizienten im Vergleich der Tabellen gemäß Fig. 4 und 5 ergibt. Bei den Kreuzkorrelationskoeffizienten drei Monate nach Implantation (Fig. 5) beträgt der minimale Korrelationskoeffizient in der Tat 0,64, wogegen sechs Wochen nach Implantation dieser minimale Koeffizient noch 0,92 beträgt. Dies deutet darauf hin, daß Morphologien, die anfänglich nach der Implantation noch sehr vergleichbar bei verschiedenen Patienten sind, im Laufe der Zeit auseinanderdriften.

Aufgrund der hohen Korrelationswerte sowohl des Tages- als auch Nacht-Mittelwertes, wie sie sich aus den Fig. 4 und 5 ergeben, folgt, daß trotz der hohen Standardabweichung diese Meßkurven als Basis für Auswertungen und Einstellungen von Betriebsparametern bei Herzschrittmachern herangezogen werden können, wie dies das erfindungsgemäße Betriebsverfahren umsetzt. So kann ein Tag-/Nacht-Wechsel (oder umgekehrt) im Sinne des erfindungsgemäßen Betriebsverfahrens auf der Basis der morphologischen Signalverläufe der intrakardialen Impedanz unter laufender Berechnung der gespeicherten Parameterdaten in Form der beispielsweise in den Fig. 1 bis 3 angegebenen Meßpunkte ermittelt werden. Dazu werden fortlaufend die Korrelationswerte während eines bestimmten Intervalles errechnet, das mit der Meßzeit fortschreitet. Befinden wir uns im Tages- oder Nachtzyklus, so werden bei dieser laufenden Berechnung ständig Autokorrelationswerte bestimmt, die entsprechend den Tabellen gemäß Fig. 4 und 5 nahe 1,0 liegen. Sobald das Auswerteintervall in einen Tag-/Nacht-Wechsel (oder umgekehrt) hineinläuft, geht aufgrund der Änderung des morphologischen Signalverlaufs (s. Unterschied zwischen Kurvenverlauf in Fig. 2 und 3) der Autokorrelationswert nach unten, durchläuft ein Minimum und nähert sich wieder den hohen Werten nahe 1,0 an, wenn der Tag-/Nacht-Wechsel aus dem Auswerteintervall hinausgelaufen ist. Dieser Durchlauf eines Minimalwertes wird durch einen Vergleich der Korrelationswerte mit einem beispielsweise durch klinische Tests oder laufend aus jeweils vorhergehenden Messungen ermittelten Referenz-Korrelationswert, der im Herzschrittmacher gespeichert ist, ermittelt, wobei das Überschreiten einer definierten Abweichung zwischen diesen beiden Werten das Vorliegen des signifikanten Ereignisses in Form eines Tag-/Nacht-Wechsels indiziert und vom Herzschrittmacher in entsprechende Änderungen bei den Schrittmacherparametem (z. B. Änderung der Reizschwellen, der Rate usw.) umgesetzt werden kann.

Die vorstehend beschriebene Auswertung der morphologischen Signalverläufe der intrakardialen Impedanz können auch zur Erkennung von oder Vorwarnung vor pathologischen Zuständen des Herzens verwendet werden. Beispielsweise führen sogenannte Extrasystolen, also zusätzliche, außerhalb des regulären Herztaktes liegende ventrikuläre Kontraktionen des Herzens oder Ischämie-Phasen, zu Signalverläufen, die eine sehr geringe Korrelation zu denjenigen gesunder Patienten aufweisen. Insoweit können Korrelationswerte, wie z. B. die Standardabweichung beim Tagesmittelwert- bzw. Nachtmittelwertsignal, mit denen gesunder Patienten verglichen werden. Liegt durch hohe Schwankungen des morphologischen Signalverlaufs die Standardabweichung hoch, so wird eine Überschreitung einer definierten Abweichung von einem Referenz-Korrelationswert für die Standardabweichung vom Auswerteprogramm des Herzschrittmachers erkannt und kann in signifikanter Weise als Vorliegen eines möglicherweise pathologischen Zustandes des Herzens erfaßt werden. Das kardiologische Geräteimplantat kann entsprechend ein Warnsignal setzen, das telemetrisch beispielsweise zu einer Überwachungszentrale übertragbar ist oder sein Betriebsprogramm verändern. Letztere kann entsprechende Maßnahmen zur Patientenfürsorge treffen.

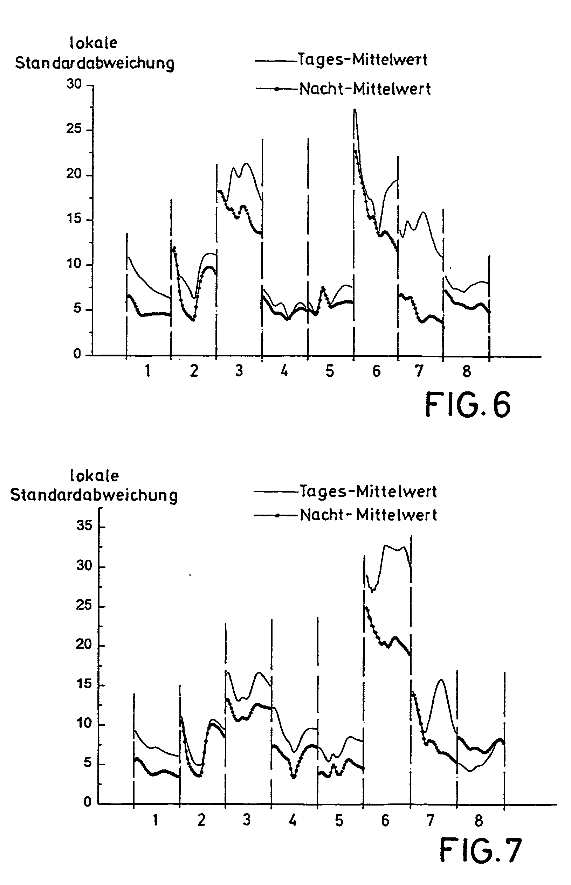

In Fig. 6 und 7 sind die Standardabweichungen des Tages- und Nachtmittelwertes in willkürlichen Einheiten für die verschiedenen Patienten (Nr. 1 bis 8 auf der X-Achse) aufgetragen. Für jeden Patienten spiegelt die Kurve den Verlauf der Standardabweichung während des Meßzeitfensters wieder. Wie Fig. 6 erkennen läßt, ist bei den Patienten Nr. 3 und 6 die Standardabweichung signifikant erhöht, was auf Probleme schließen läßt. Fig. 7 läßt erkennen, daß während des Zeitraums von sechs Wochen nach der Implantation (Fig. 6) bis drei Monate nach der Implantation (Fig. 7) des Herzschrittmachers sich beim Patienten Nr. 3 die Standardabweichung deutlich verringert hat, was auf eine Annäherung dieses Patientens an "Normalbedingungen" schließen läßt.

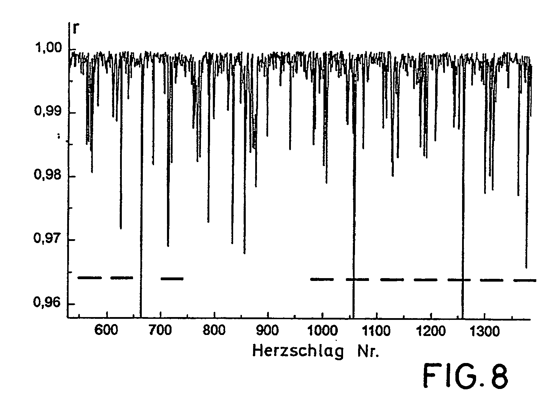

Schließlich können auf der Basis der gemessenen und gespeicherten Parameterdaten alternativ auch periodisch wiederkehrende Einflüsse auf die Herztätigkeit mit niedrigen Frequenzen beispielsweise im Bereich von 0,1 Hz oder 0,25 Hz detektiert werden. Ein mit einem entsprechenden Betriebsverfahren ausgerüsteter Herzschrittmacher wird den zeitlichen Verlauf der Abweichung der Einzelsignalverläufe von einem gemittelten Durchschnittssignalverlauf oder einem vorgegebenen Referenzsignalverlauf erfassen und mit Mitteln der Spektralanalyse, wie z. B. einer Fast-Fourier-Transformation, analysieren, so daß regelmäßig wiederkehrende Abweichungen in den Signalverläufen mit niedrigen Repetitionsfrequenzen detektierbar sind. So ist in der beigefügten Fig. 8 der Korrelationskoeffizient r auf der y-Achse in Abhängigkeit der Zeit (hier repräsentiert durch aufeinanderfolgende Herzschläge der laufenden Nummern unter 600 bis über 1300) auf der x-Achse aufgetragen. In diesem Diagramm ist erkennbar, daß Ausschläge des Korrelationskoeffizienten nach unten in regelmäßigen Abständen auftreten, obwohl die absolute Veränderung des Korrelationskoeffizienten nur bei ca. 0,02 liegt. Das in Fig. 8 gezeigten Kurvenmuster ähnelt sehr stark dem Verhalten der Respirationsrate unter Einfluß des sogenannten Cheyne-Stokes-Syndrom. Bei dieser Krankheit findet die Atmung mit einer bestimmten Basisfrequenz statt, wird jedoch in regulären Intervallen um eine gleichbleibende Spanne herabgesetzt. Insoweit können aufgrund der Messung und Auswertung der Signalmorphologie der intrakardialen Impedanz des Herzens mit Hilfe des Herzschrittmachers auch über Herz-fremde, insbesondere pulmonale Einflüsse und Symptome, entsprechende Erkenntnisse gewonnen und beispielsweise in Form eines Warnsignals gemeldet werden.

Ähnliche Einflüsse wie in Fig. 5 können im übrigen beispielsweise mit einer Frequenz von 0,1 Hz auftreten. Diese stehen mit der vagalen bzw. sympathischen Nerventätigkeit im Zusammenhang mit dem Blutdruck in Verbindung. Ein erhöhter vagaler Einfluß, der aus Schwankungen des Korrelationskoeffizienten mit einer Frequenz von 0,1 Hz zu vermuten wäre, ließe darauf schließen, daß die Herzrate für die herrschenden physiologischen Gegebenheiten überhöht ist. Dies kann unter Umständen auf Ischämie-Phasen oder ungeeignete Herzschrittmacher-Einstellungen hinweisen. Ein entsprechend ausgewertetes Signal kann also als Referenz herangezogen werden, die Herzschrittmacher-Einstellungen durch Änderung des Betriebsprogramms selbttätig zu optimieren. Nicht zuletzt sind auch rejektionsdiagnostische Auswerteergebnisse nach Organ-/Herz-Transplantationen ermittelbar.

## Patentansprüche

1. Kardiologisches Geräteimplantat, insbesondere Herzschrittmacher, gesteuert durch ein Betriebsverfahren mit folgenden Verfahrensschritten zur Detektion eines signifikanten Ereignisses oder Zustandes, dem ein Patient unterworfen ist:
- Messung der intrakardialen Impedanz des Herzens,
- Erfassung des morphologischen Signalverlaufs der Impedanz anhand mindestens eines morphologisch repräsentativen Parameters des Signalverlaufs,
- laufende Speicherung des Parameters aus einer definierten Meßperiode,
- Berechnung eines Korrelationswertes für den gespeicherten Parameter, und
- Vergleichen des Korrelationswertes mit einem Referenz-Korrelationswert, wobei das Überschreiten einer definierten Abweichung zwischen Korrelations- und Referenz-Korrelationswerten das Vorliegen des signifikanten Ereignisses oder Zustandes indiziert,
**dadurch gekennzeichnet, daß** die gespeicherten Korrelationswerte, insbesondere Autokorrelationswerte, derart ausgewertet werden, daß das Durchlaufen eines Minimum als Tag-/Nacht- oder Nacht-/Tag-Wechsel erkannt und das Betriebsprogramm des kardiologischen Geräteimplantates entsprechend eingestellt wird.

2. Kardiologisches Geräteimplantat, insbesondere Herzschrittmacher, nach Anspruch 1, **dadurch gekennzeichnet, daß** zur Erfassung des morphologischen Signalverlaufs der Impedanz eine Vielzahl von Meßpunkten als Parameterdaten während einer Herzperiode aufgezeichnet werden.

3. Kardiologisches Geräteimplantat, insbesondere Herzschrittmacher, nach Anspruch 1, **dadurch gekennzeichnet, daß** zwei die Peak-to-peak-Amplitude des Signalverlaufs wiedergebende Parameterdaten aufgezeichnet werden.

4. Kardiologisches Geräteimplantat, insbesondere Herzschrittmacher, nach Anspruch 2 oder 3, **dadurch gekennzeichnet, daß** als Korrelationswert ein Autokorrelationswert der gespeicherten Parameterdaten bestimmt wird.

5. Kardiologisches Geräteimplantat, insbesondere Herzschrittmacher, nach Anspruch 2 oder 3, **dadurch gekennzeichnet, daß** als Korrelationswert die quadratische oder Standardabweichung der Parameterdaten bestimmt wird.

6. Kardiologisches Geräteimplantat, insbesondere Herzschrittmacher, nach einem der Ansprüche 1 bis 5, **dadurch gekennzeichnet, daß** die Korrelationswerte fortlaufend berechnet und mit den Referenz-Korrelationswerten verglichen werden.

7. Kardiologisches Geräteimplantat, insbesondere Herzschrittmacher, nach einem der Ansprüche 1 bis 4, **dadurch gekennzeichnet, daß** die während eines bestimmten Intervalles errechneten Korrelationswerte laufend zu Auswertungszwecken gespeichert werden.

8. Kardiologisches Geräteimplantat, insbesondere Herzschrittmacher, nach einem der Ansprüche 1 bis 7, **dadurch gekennzeichnet, daß** die gespeicherten Korrelationswerte, insbesondere Autokorrelationswerte, derart ausgewertet werden, daß ein Überschreiten der definierten Abweichung von Referenz-Korrelationswerten in signifikanter Weise als Vorliegen eines möglicherweise pathologischen Zustandes des Herzens erkannt wird und das kardiologische Geräteimplantat ein entsprechendes Warnsignal setzt.

9. Kardiologisches Geräteimplantat, insbesondere Herzschrittmacher, nach einem der Ansprüche 1 bis 8, **dadurch gekennzeichnet, daß** die Referenz-Korrelationswerte abhängig von der Implantierungsdauer des Implantats einstellbar sind

10. Kardiologisches Geräteimplantat, insbesondere Herzschrittmacher, nach einem der Ansprüche 1 bis 9, **dadurch gekennzeichnet, daß** auf der Basis der Parameterdaten für die intrakardiale Impedanz der zeitliche Verlauf der Abweichung deren Einzelsignalverläufe von einem gemittelten Durchschnittssignalverlauf oder einem vorgegebenen Referenzsignalverlauf oder voneinander erfaßt und mit Mitteln der Spektralanalyse derart analysiert wird, daß regelmäßig wiederkehrende Abweichungen mit Frequenzen, die mit physiologischen Funktionen des Körpers gekoppelt sind, detektierbar sind.

11. Kardiologisches Geräteimplantat, insbesondere Herzschrittmacher, nach einem der Ansprüche 1 bis 10, **gekennzeichnet durch** eine Auswahlmöglichkeit zwischen verschiedenen Auswerteverfahren und -parametern.

12. Kardiologisches Geräteimplantat, insbesondere Herzschrittmacher, nach Anspruch 11, **dadurch gekennzeichnet, daß** die Auswahl automatisch in Abhängigkeit des vorhergehenden Auswerteergebnisses oder durch Vorgabe des Betriebsprogrammes des Geräteimplantats erfolgt.

## Claims

1. A cardiological device implant, in particular a cardiac pacemaker, controlled by an operating method having the following method steps for detecting a significant event or state, to which a patient is subjected:
- measuring the intracardial impedance of the heart,
- detecting the morphological signal curve of the impedance on the basis of at least one morphologically representative parameter of the signal curve,
- continuosly storing of the parameter from a defined measurement period,
- calculating a correlation value for the stored parameter, and
- comparing the correlation value to a reference correlation value, wherein the exceeding of a defined deviation between correlation value and reference correlation value indicates the presence of the significant event or state,
**characterized in that** the stored correlation values, in particular autocorrelation values, are analyzed in such a way that passage through a minimum is recognized as a day/night or night/day change and the operating program of the cardiological device implant is adjusted accordingly.

2. The cardiological device implant, in particular cardiac pacemaker, according to Claim 1, **characterized in that** multiple measured points are recorded as parameter data during a heart period to acquire the morphological signal curve of the impedance.

3. The cardiological device implant, in particular cardiac pacemaker, according to Claim 1, **characterized in that** two sets of parameter data reflecting the peak-to-peak amplitude of the signal curve are recorded.

4. The cardiological device implant, in particular cardiac pacemaker, according to Claim 2 or 3, **characterized in that** an autocorrelation value of the stored parameter data is determined as the correlation value.

5. The cardiological device implant, in particular cardiac pacemaker, according to Claim 2 or 3, **characterized in that** the root mean square or standard deviation of the parameter data is determined as the correlation value.

6. The cardiological device implant, in particular cardiac pacemaker, according to one of Claims 1 through 5, **characterized in that** the correlation values are continuously calculated and compared to the reference correlation values.

7. The cardiological device implant, in particular cardiac pacemaker, according to one of Claims 1 through 4, **characterized in that** the correlation values calculated during a specific interval are continuously stored for analysis purposes.

8. The cardiological device implant, in particular cardiac pacemaker, according to one of Claims 1 through 7, **characterized in that** the stored correlation values, in particular autocorrelation values, are analyzed in such a way that exceeding the defined deviation of reference correlation values significantly is recognized as the presence of a possibly pathological state of the heart, and the cardiological device implant sets a corresponding warning signal.

9. The cardiological device implant, in particular cardiac pacemaker, according to one of Claims 1 through 8, **characterized in that** the reference correlation values are adjustable as a function of the implantation time of the implant.

10. The cardiological device implant, in particular cardiac pacemaker, according to one of Claims 1 through 9, **characterized in that** the time curve of the deviation of its individual signal curves from an averaged average signal curve or a predefined reference signal curve or from one another are detected on the basis of the parameter data for the intracardial impedance and analyzed using means for spectral analysis in such a way that regularly repeating deviations having frequencies which are coupled to physiological functions of the body are detectable.

11. The cardiological device implant, in particular cardiac pacemaker, according to one of Claims 1 through 10, **characterized by** a selection possibility between various analysis methods and parameters.

12. The cardiological device implant, in particular cardiac pacemaker, according to Claim 11, **characterized in that** the selection occurs automatically as a function of the preceding analysis result or by presetting the operating program of the device implant.

## Revendications

1. Implant d'appareil cardiologique, en particulier pacemaker, commandé par un procédé d'exploitation avec les étapes de procédé suivantes pour la détection d'un événement ou d'un état significatif, auquel est soumis un patient :
- mesure de l'impédance intracardiaque du coeur,
- enregistrement de la courbe de signal morphologique de l'impédance à l'aide d'au moins un paramètre représentatif au plan morphologique de la courbe de signal,
- stockage continu du paramètre à partir d'une période de mesure définie,
- calcul d'une valeur de corrélation pour le paramètre stocké et
- comparaison de la valeur de corrélation avec une valeur de corrélation de référence, le dépassement d'un écart défini entre des valeurs de corrélation et des valeurs de corrélation de référence indiquant la présence de l'événement ou de l'état significatif, **caractérisée en ce que** les valeurs de corrélation stockées, en particulier des valeurs d'autocorrélation, sont analysées de telle sorte que le passage par un minimum est reconnu comme passage jour/nuit ou nuit/jour et le programme d'exploitation de l'implant d'appareil cardiologique est réglé en conséquence.

2. Implant d'appareil cardiologique, en particulier pacemaker, selon la revendication 1, **caractérisé en ce qu'**une pluralité de points de mesure est enregistrée comme données de paramètre pendant une période cardiaque pour l'enregistrement de la courbe de signal morphologique.

3. Implant d'appareil cardiologique, en particulier pacemaker, selon la revendication 1, **caractérisé en ce que** deux données de paramètre restituant respectivement l'amplitude pic à pic de la courbe de signal sont enregistrées.

4. Implant d'appareil cardiologique, en particulier pacemaker, selon la revendication 2 ou 3, **caractérisé en ce qu'**une valeur d'autocorrélation des données de paramètre stockées est déterminée comme valeur de corrélation.

5. Implant d'appareil cardiologique, en particulier pacemaker, selon la revendication 2 ou 3, **caractérisé en ce que** l'écart quadratique ou l'écart-type des données de paramètre est déterminé comme valeur de corrélation.

6. Implant d'appareil cardiologique, en particulier pacemaker, selon l'une quelconque des revendications 1 à 5, **caractérisé en ce que** les valeurs de corrélation sont calculées de façon permanentes et comparées avec les valeurs de corrélation de référence.

7. Implant d'appareil cardiologie, en particulier pacemaker, selon l'une quelconque des revendications 1 à 4, **caractérisé en ce que** les valeurs de corrélation calculées pendant une période définie sont mémorisées de façon permanente pour les besoins d'analyse.

8. Implant d'appareil cardiologique, en particulier pacemaker, selon l'une quelconque des revendications 1 à 7, **caractérisé en ce que** les valeurs de corrélation stockées, en particulier les valeurs d'autocorrélation, sont analysées de telle sorte qu'un dépassement de façon significative de l'écart défini par rapport à des valeurs de corrélation de référence est détecté comme présence d'un état éventuellement pathologique du coeur et l'implant d'appareil cardiologique émet un signal d'avertissement approprié.

9. Implant d'appareil cardiologie, en particulier pacemaker, selon l'une quelconque des revendications 1 à 8, **caractérisé en ce que** les valeurs de corrélation de référence peuvent être réglées en fonction de la durée d'implantation de l'implant.

10. Implant d'appareil cardiologie, en particulier pacemaker, selon l'une quelconque des revendications 1 à 9, **caractérisé en ce que**, sur la base des données de paramètre pour l'impédance intracardiaque, on enregistre la variation dans le temps de l'écart de leurs courbes de signal individuel par rapport à une courbe moyenne de signal moyen ou par rapport à une courbe prédéfinie de signal de référence ou les unes par rapport aux autres et on l'analyse avec des moyens d'analyse spectrale de telle sorte que des écarts récurrents de façon régulière sont détectés avec des fréquences qui sont couplées à des fonctions physiologiques du corps.

11. Implant d'appareil cardiologique, en particulier pacemaker, selon l'une quelconque des revendications 1 à 10, **caractérisé par** une possibilité de choix entre différents procédés d'analyse et paramètres d'analyse.

12. Implant d'appareil cardiologique, en particulier pacemaker, selon la revendication 11, **caractérisé en ce que** le choix s'effectue automatiquement en fonction du résultat d'analyse précédent ou par spécification du programme d'exploitation de l'implant d'appareil.
